# EUROPEAN PATENT APPLICATION

(11) **EP 2 835 106 A1**
(43) Date of publication of application: **11.02.2015**
(21) Application number: 14180183.7
(22) Date of filing: 07.08.2014
(51) Int. Cl.: A61B 17/3203, A61B 19/00, A61B 19/02

(54) **Medical instrument holding apparatus**

(30) Priority: 09.08.2013 JP 2013166012
(71) Applicant: Seiko Epson Corporation, Shinjuku-ku Tokyo (JP)
(72) Inventor: Sekino, Hirokazu, Nagano, 392-8502 (JP); Hirabayashi, Atsuya, Nagano, 392-8502 (JP); Kawakami, Daisuke, Nagano, 392-8502 (JP)
(74) Representative: Hoffmann Eitle

(57) **Abstract**

A medical instrument holding apparatus (stand) that holds a medical instrument having a functional unit, includes: an instrument housing unit capable of housing at least a part of the instrument; and a coordination unit which coordinates with the functional unit when at least a part of the instrument is housed in the instrument housing unit. Thus, as the medical instrument is housed in this medical instrument holding apparatus, coordination between the two is possible.

## Description

### BACKGROUND

### 1. Technical Field

The present invention relates to a medical instrument holding apparatus which holds a medical instrument having a functional unit inside.

### 2. Related Art

Recently, various medical instruments such as electric surgical knife are proposed. There is, for example, a liquid ejection device which ejects a liquid such as water or physiological saline solution in a pulse form from a nozzle to excise or incise a living tissue (JP-A-2008-82202). If this liquid ejection device is used for a surgical operation, the operation can be carried out without causing heat damage to the living tissue or damaging tissues of blood vessels, nerves and the like. Such a liquid ejection device has a medical instrument which the operator holds in the hand (hereinafter referred to as a handpiece), and a supply device which supplies the liquid to the handpiece.

When carrying out a surgical operation or the like, the operator holds the handpiece in the hand to use the handpiece but may replace the handpiece with another surgical instrument such as electric surgical knife. In such a case, the handpiece is left as it is in the peripheral space of the operating table or suspended on a metal fitting.

Thus, a stand to hold a medical instrument such as a handpiece upright may be considered. However, since a functional unit to realize various functions is assembled in the medical instrument, simply preparing the stand to hold the medical instrument upright does not provide a sufficient solution. The medical instrument has various constraints due to the functions thereof. For example, in an operating theater, no contamination from the patient is allowed and therefore basically the instrument used for the operation must not be reused. Even a stand must not be reused once the medical instrument touches the stand. However, how to determine whether the stand is already used or not is not considered so far. Similarly, there are many unresolved problems with the holding apparatus to hold such a medical instrument.

Such problems are not limited only to the handpiece for the liquid ejection device and the holding apparatus for the handpiece but can also be applied to other medical instruments such as electric surgical knife and laser knife. In the case of a device that ejects a liquid, there are further problems due to the ejection of the liquid. For example, in the case of the handpiece of a liquid ejection device, since a valve or the like is not provided at an ejection port (nozzle) that ejects the liquid, the liquid in the channel inside the handpiece may flow out from the distal end of the nozzle if the handpiece is left unused for a long time. It can also be considered that if the handpiece is left unused for a long time, the substance attached to the distal end of the handpiece may dry and solidify, thus generating the need to clean the distal end every time the handpiece is used subsequently.

Moreover, with respect to the medical instrument holding apparatus, there are problems such as miniaturization of the device, cost reduction, resource saving, easier manufacturing, and improved user-friendliness.

### SUMMARY

An advantage of some aspects of the invention is that a medical instrument holding apparatus used for a medical instrument that can overcome at least a part of the foregoing problems of the related-art techniques is provided.

The invention can be implemented as the following forms.

(1) An aspect of the invention provides a medical instrument holding apparatus that holds a medical instrument having a functional unit. The medical instrument holding apparatus includes: an instrument housing unit capable of housing at least a part of the instrument; and a coordination unit which coordinates with the functional unit when at least a part of the instrument is housed in the instrument housing unit.

According to this medical instrument holding apparatus, when at least a part of the medical instrument is housed therein, the coordination unit coordinates with the function realized in the instrument by the functional unit of the instrument. Therefore, the function of the medical instrument can be exhibited sufficiently.

(2) In the medical instrument holding apparatus, the function of the instrument may be a function of ejecting a liquid outside from an ejection portion provided on the instrument. The instrument housing unit may house at least the ejection portion. In this medical instrument holding apparatus, coordination with the function of ejecting the liquid in the medical instrument can be realized.

(3) For example, the coordination unit may be a cleaning device which cleans at least the ejection portion of the liquid of the instrument housed in the instrument housing unit. Thus, when the ejection portion of the medical instrument is housed in the medical instrument holding apparatus, the ejection portion can be cleaned and the instrument can be kept clean during the operation.

(4) Alternatively, the coordination unit may include a liquid discharge device which discharges the liquid from inside the instrument housing unit. Thus, in the medical instrument holding apparatus, the liquid that flows out can be discharged outside, in coordination with the function of the medical instrument that ejects the liquid.

(5) Also, the coordination unit may include a high molecular polymer which adsorbs the liquid leaking out of the instrument in the instrument housing unit. Since the high molecular polymer can adsorb a much greater amount of liquid than its own volume, the leakage of the liquid from the instrument can be dealt with without having to provide a mechanism to discharge leaking liquid. As the medical instrument is disposable in principle, the liquid leaking out in the use for a single operation can be easily adsorbed.

(6) In the medical instrument holding apparatus, the coordination unit may include a signal output unit which outputs a signal outside when the medical instrument is housed in the instrument housing unit. In this medical instrument holding apparatus, since a signal is outputted outside when the medical instrument is housed in the instrument housing unit, various measures can be taken, using this signal. For example, in the cleaning, a cleaning solution can be supplied from outside and discharged outside. Alternatively, the signal can be used to monitor the state of the medical instrument outside.

(7) The coordination unit may include an actuation unit which causes a recognition device provided on the side of the medical instrument housed in the instrument housing unit to recognize that a part of the instrument is housed in the instrument housing unit. In this medical instrument holding apparatus, the instrument side can be made to recognize that a part of the instrument is housed in the instrument housing unit, pretreatment for use or the like can be carried out in the state where the instrument is housed in the instrument housing unit. The pretreatment can be, for example, processing to fill the inside of an instrument that ejects a liquid, with the liquid in advance, or processing to extract the air accumulated in a pipe before use. Of course, in other instruments such as electric surgical knife, processing such as turning on electricity or adjusting voltage that is needed or confirmed before use may be carried out.

(8) In the medical instrument holding apparatus, the coordination unit may include a data unit which communicates data that causes at least one of a type of the medical instrument holding apparatus and an individual to be recognized, with the instrument housed in the instrument housing unit. In this medical instrument holding apparatus, since the data is communicated, whether the medical instrument and the medical instrument holding apparatus are of a correct combination or not, whether inappropriate reuse is avoided or not, and the like can be confirmed.

(9) In the medical instrument holding apparatus, the coordination unit may include a member which carries out at least one of heating and cooling of the instrument housed in the instrument housing unit. Thus, the instrument can be heated or cooled according to need.

(10) Since a medical instrument holding apparatus that is contaminated in a surgical operation or the like must not be reused, the medical instrument holding apparatus may include a film which covers an inlet port of the instrument housing unit before the use of the medical instrument holding apparatus, so that it can be visually recognized that the apparatus is a new medical instrument holding apparatus that is not reused. By disinfecting or sterilizing the inside of the film, the medical instrument holding apparatus can be kept clean until its use.

(11) In view of this, the medical instrument holding apparatus may be sterilized and packed with the medical instrument. Thus, the medical instrument holding apparatus and the medical instrument can be kept clean before the operation. Moreover, it is possible to clarify that the medical instrument holding apparatus is unused. Also, the part of the medical instrument in the state of being housed in the instrument housing unit may be placed in a bag and then sterilized. Moreover, the medical instrument holding apparatus may be singly placed in a bag and disinfected.

(12) The medical instrument holding apparatus may include a replacement housing unit which houses a part including at least a distal end of a replaceable replacement member provided on the medical instrument, in order to replace the replacement member, and a detachment unit which detaches the replacement member housed in the replacement housing unit from the instrument. In this medical instrument holding apparatus, the replacement member provided at a distal end of a medical instrument can be easily detached, using the medical instrument holding apparatus. Particularly, since the operator need not touch other members for the replacement, the replacement member can be detached more safely.

(13) Here, the medical instrument holding apparatus may further include a new replacement member installed on the instrument instead of the detached replacement member, and a replacement member installing unit which houses the new replacement member in such a way that the new replacement member can be installed on the instrument. Thus, the replacement member can be replaced with a new member, using the medical instrument holding apparatus. In this case, since the operator need not touch other members, the replacement member can be replaced more safely.

(14) When at least a part of a liquid ejection device is housed as a medical instrument in the medical instrument holding apparatus, with the function of the coordination unit, a predetermined discharge operation to discharge a liquid from inside a liquid chamber can be executed on the side of the liquid ejection device. Since the gas inside the liquid chamber is discharged with the liquid, the operator need not carry out any work to discharge the gas from inside the liquid chamber. Therefore, the burden on the operator can be reduced. Moreover, when the operator is not holding the liquid ejection device (when ejection of the liquid is not carried out), the liquid ejection device may be held in the medical instrument holding apparatus, and a predetermined discharge operation to discharge the liquid from inside the liquid chamber may be carried out by the time when the operator holds the liquid ejection device to eject the liquid next time. Consequently, the inside of the liquid chamber can be constantly kept in the gas-discharged state. The operator can immediately carry out ejection of the liquid when the operator wants to use the liquid ejection device. Such a liquid discharge operation may be carried out continuously or may be carried out intermittently at a certain rate such as every several seconds or every several ten seconds.

(15) When the liquid ejection device is first held in the medical instrument holding apparatus, a liquid supply unit which supplies the liquid to the liquid ejection device may be operated for a predetermined time. Thus, by properly setting the predetermined time, it is possible to carry out initial filling of the liquid when the liquid ejection device is held in the medical instrument holding apparatus, and thus make the liquid ejection device immediately available for use.

(16) Moreover, in the medical instrument holding apparatus, a unit which sucks the liquid from a nozzle of the liquid ejection device may be provided. If the suction unit is thus provided in the medical instrument holding apparatus, the liquid can be powerfully sucked out of the nozzle of the liquid ejection device. Consequently, even if the nozzle is clogged, the clogging can be solved.

### BRIEF DESCRIPTION OF THE DRAWINGS

The invention will be described with reference to the accompanying drawings, wherein like numbers reference like elements.
FIG. 1 is an explanatory view showing the state where a liquid ejection device is installed on a stand that is a medical instrument holding apparatus according to a first embodiment of the invention.
FIG. 2 is an explanatory view showing the schematic configuration of the liquid ejection device of the embodiment and the flow of a liquid.
FIG. 3 is a cross-sectional view showing the detailed structure of the stand.
FIGS. 4A and 4B are explanatory views showing the operation in which the liquid ejection device ejects the liquid.
FIG. 5 is a flowchart showing liquid discharge processing carried out by a control unit.
FIGS. 6A to 6C are explanatory views showing the state where a first switch turns on when the liquid ejection device is set on the stand.
FIG. 7 is an explanatory view roughly showing the configuration of a liquid ejection device and a stand according to a second embodiment.
FIG. 8 is a cross-sectional view showing the structure of a stand according to a third embodiment.
FIG. 9 is a cross-sectional view showing a modification of the third embodiment.
FIG. 10 is a cross-sectional explanatory view showing the structure of a stand which carries out cleaning, as a fourth embodiment.
FIG. 11 is a cross-sectional explanatory view showing the structure of a stand capable of heating and cooling, as a fifth embodiment.
FIG. 12 is a cross-sectional explanatory view showing the structure of a stand according to a sixth embodiment.
FIG. 13 is a flowchart showing how data is exchanged between the stand and a liquid ejection device according to the sixth embodiment.
FIG. 14 is a flowchart showing a modification of the sixth embodiment.
FIG. 15 is a cross-sectional explanatory view showing the configuration of a stand according to a seventh embodiment.
FIG. 16 is a cross-sectional explanatory view showing the structure of a stand according to an eighth embodiment.
FIG. 17 is an explanatory view showing the form of providing a stand and a liquid ejection device according to a ninth embodiment.
FIG. 18 shows the process of provision according to the ninth embodiment.
FIG. 19 is a cross-sectional explanatory view showing the structure of a stand according to a tenth embodiment.
FIGS. 20A and 20B are explanatory views showing the state of use of the stand according to the tenth embodiment.
FIG. 21 is an explanatory view illustrating the form of a stand according to an eleventh embodiment.
FIG. 22 is an explanatory view illustrating another embodiment.

### DESCRIPTION OF EXEMPLARY EMBODIMENTS

First to eleventh embodiments of the invention will be described. Common members are denoted by common reference numbers. A structure and control described in one embodiment can also be applied to other embodiments.

### 1 First Embodiment

### Configuration of Apparatus

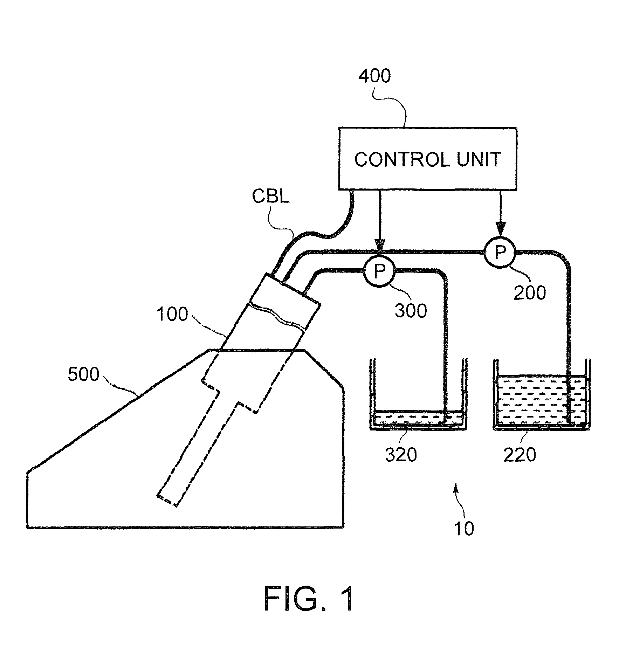

FIG. 1 is a schematic configuration view showing the state where a liquid ejection device 100 is installed on a stand 500 that is a medical instrument holding apparatus according to a first embodiment of the invention. As illustrated, in terms of electric system, the liquid ejection device 100 is connected to a control unit 400 via a cable CBL. In terms of fluid system, the liquid ejection device 100 is connected to a liquid container 220 and a drainage container 320 via tubes. The supply and discharge of a liquid is carried out by a feed pump 200 and a suction pump 300 with the operations thereof controlled by the control unit 400. The communication of signals between the liquid ejection device 100 and the control unit 400, and the supply and discharge of the liquid under the control of the control unit 400 will be described in detail later. The overall configuration including the liquid ejection device 100, the control unit 400 and the configurations to carry out the supply and discharge of the liquid is called a liquid ejection device system 10.

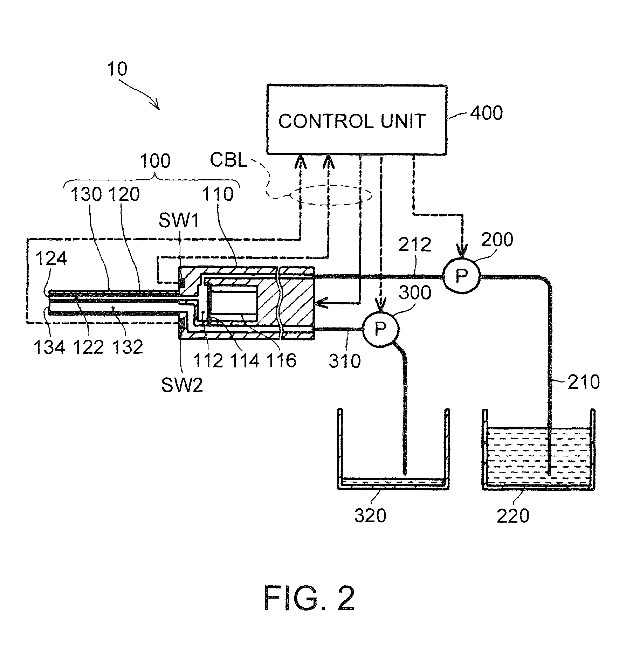

Next, the structure of the liquid ejection device system 10 will be described with reference to FIG. 2. As illustrated, the liquid ejection device system 10 is used for a surgical operation in which a liquid such as water or physiological saline solution is ejected toward a living tissue to incise or excise the living tissue. The liquid ejection device 100 forming the system 10 is a handpiece which an operator holds in the hand to eject the liquid. The feed pump 200 that supplies the liquid is connected to the liquid ejection device 100 via a second connection tube 212. A first connection tube 210 is connected to the feed pump 200 so that the liquid to be ejected by the liquid ejection device 100 can be pumped up from the liquid container 220. Also, the suction pump 300 is connected to the liquid ejection device 100 via a third connection tube 310. The liquid sucked by the suction pump 300 is discharged to the drainage container 320 via a fourth connection tube 312.

The liquid ejection device 100 roughly includes a main body unit 110, and a liquid ejection tube 120 and a suction tube 130 or the like provided in a standing manner on the main body unit 110. A nozzle 124 is formed at the distal end of the liquid ejection tube 120. The nozzle 124 is connected to a liquid chamber 112 of the main body unit 110 via an ejection channel 122 of the liquid ejection tube 120 and an internal channel of the main body unit 110. The liquid chamber 112 is connected to the feed pump 200 via the internal channel of the main body unit 110 and the second connection tube 212. The feed pump 200 is connected to the liquid container 220 via the first connection tube 210 and supplies the liquid sucked up from the liquid container 220 to the liquid chamber 112 of the liquid ejection device 100 via the second connection tube 212. The liquid chamber 112 is partly formed with a metallic diaphragm 114, and a piezoelectric element 116 is provided in the state of abutting against the diaphragm 114 from outside of the liquid chamber 112. As described in detail later, as a drive signal is applied to the piezoelectric element 116, the liquid in the liquid chamber 112 is ejected in a pulse form from the nozzle 124.

The suction tube 130 is provided on the outside of the liquid ejection tube 120. The suction tube 130 is a tube with a slightly larger diameter than the liquid ejection tube 120 and has the liquid ejection tube 120 housed therein over a predetermined length. A suction opening 134 is formed at the distal end of the suction tube 130. The suction opening 134 is connected to the suction pump 300 via a suction channel 132 of the suction tube 130, the internal channel of the main body unit 110 and the third connection tube 310. While the liquid is ejected to a living tissue from the nozzle 124, the suction pump 300 is driven to suck the liquid accumulated at the surgical site from the suction opening 134 via the suction channel 132.

In the liquid ejection device 100 of this example, two switches (first switch SW1, second switch SW2) are provided on the distal end side of the main body unit 110, that is, at end parts on the side where the liquid ejection tube 120 and the suction tube 130 are provided. Detection signals are outputted to the control unit 400 from the first switch SW1 and the second switch SW2 so that the control unit 400 can grasp the states of these switches. The reason for providing the first switch SW1 and the second switch SW2 will be described later.

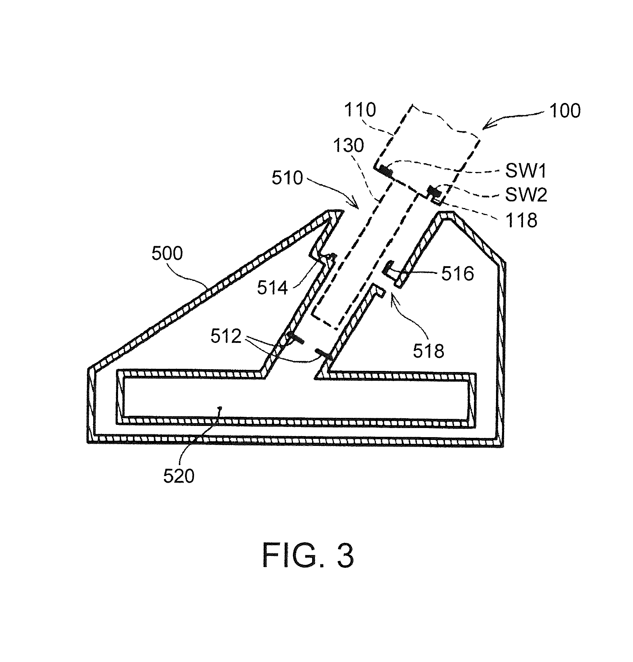

FIG. 3 is a cross-sectional view showing the detailed structure of the stand 500 of this example. In FIG. 3, the liquid ejection device 100 in the course of being set on the stand 500 is indicated by dashed lines.

As illustrated, inside the stand 500 of this example, a housing passage 510 is formed in which the suction tube 130 and a part of the main body unit 110 of the liquid ejection device 100 are housed. Below the housing passage 510 (in the interior), a space (liquid storage section 520) for storing the liquid flowing out of the nozzle 124 of the liquid ejection device 100 is formed. A rubber seal portion 512 is provided on the inner wall of the portion where the suction tube 130 is inserted, of the housing passage 510. As the suction tube 130 is inserted, the gap between the outer periphery of the suction tube 130 and the housing passage 510 is closed by the seal portion 512, thus sealing the liquid storage section 520.

In the portion where the main body unit 110 is inserted, of the housing passage 510, a protrusion 514 is provided at the position corresponding to the first switch SW1 of the liquid ejection device 100. A pin 516 is provided at the position corresponding to the second switch SW2 of the liquid ejection device 100. The pin 516 is connected to the inner wall of the housing passage 510 via a thin connection member. Moreover, at a position below the position where the pin 516 is connected, a penetrating portion 518 where the inner wall of the housing passage 510 is penetrated is provided. At the position where the main body unit 110 faces the distal end of the pin 516, of the liquid ejection device 100, a communication path 118 with substantially the same diameter as the pin 516 is provided. The second switch SW2 is provided in the interior of the communication path 118.

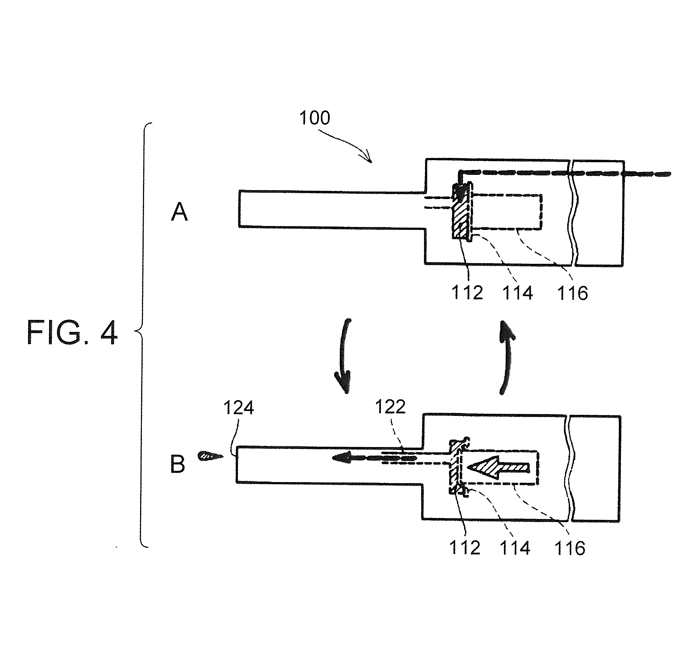

Next, the principle of the operation in which the liquid ejection device 100 ejects the liquid from the nozzle 124 will be described. As shown in FIG. 4A, as the feed pump 200 operates, the liquid chamber 112 is filled with the liquid supplied from the feed pump 200. In this state, the piezoelectric element 116 is not driven and the liquid is not ejected from the nozzle 124. In FIG. 4A, the shading of the liquid chamber 112 indicates that the liquid chamber 112 is filled with the liquid.

Subsequently, a drive voltage is applied to the piezoelectric element 116. The piezoelectric element 116 then deforms in an expanding direction, deforming the diaphragm 114 to reduce the volume of the liquid chamber 112. Consequently, the liquid in the liquid chamber 112 is pressurized and ejected from the nozzle 124 via the ejection channel 122, as shown in FIG. 4B. The deformation of the piezoelectric element 116 completes after the piezoelectric element 116 expands to a predetermined length in a short time. Therefore, as an amount of the liquid equivalent to the amount of reduction in the volume of the liquid chamber 112 due to the deformation of the piezoelectric element 116 is ejected from the nozzle 124, the ejection of the liquid stops. Thus, the ejection of the liquid from the nozzle 124 is in a pulse form.

As the liquid in the pulse form is thus ejected, the voltage applied to the piezoelectric element 116 is eliminated. The deformed piezoelectric element 116 then returns to the original length, causing the reduced volume of the liquid chamber 112 to return to the original volume. With these movements, the liquid is supplied to the liquid chamber 112 from the feed pump 200 and consequently the liquid chamber 112 returns to the state shown in FIG. 4A, that is, the state before the piezoelectric element 116 is driven. As a drive voltage is applied to the piezoelectric element 116 again in this state, the piezoelectric element 116 deforms as shown in FIG. 4B and the liquid in the liquid chamber 112 is ejected in a pulse form from the nozzle 124. In the liquid ejection device system 10, since the control unit 400 applies a pulse voltage with a predetermined frequency to the piezoelectric element 116, the liquid is ejected from the nozzle 124 in a pulse form at a repetition rate corresponding to the frequency of the applied voltage. The applied voltage has characteristics of short voltage application time (rising time) and long voltage elimination time (falling time).

The above operation is an operation in the case where the operator actually carries out a surgical operation or the like, holding the liquid ejection device 100 as a handpiece. Meanwhile, in the liquid ejection device 100, the liquid chamber 112 needs to be filled with the liquid before the use of the device. Also, if air bubbles are accumulated in the liquid chamber 112 or the like, the air bubbles need to be eliminated because the air bubbles may obstruct compression and ejection of the liquid with the diaphragm 114. The former is called an initial filling operation, whereas the latter is called an air bubble discharge operation. In this embodiment, these operations can be carried out using the stand 500. Hereinafter, this feature will be described.

### Liquid Discharge Processing

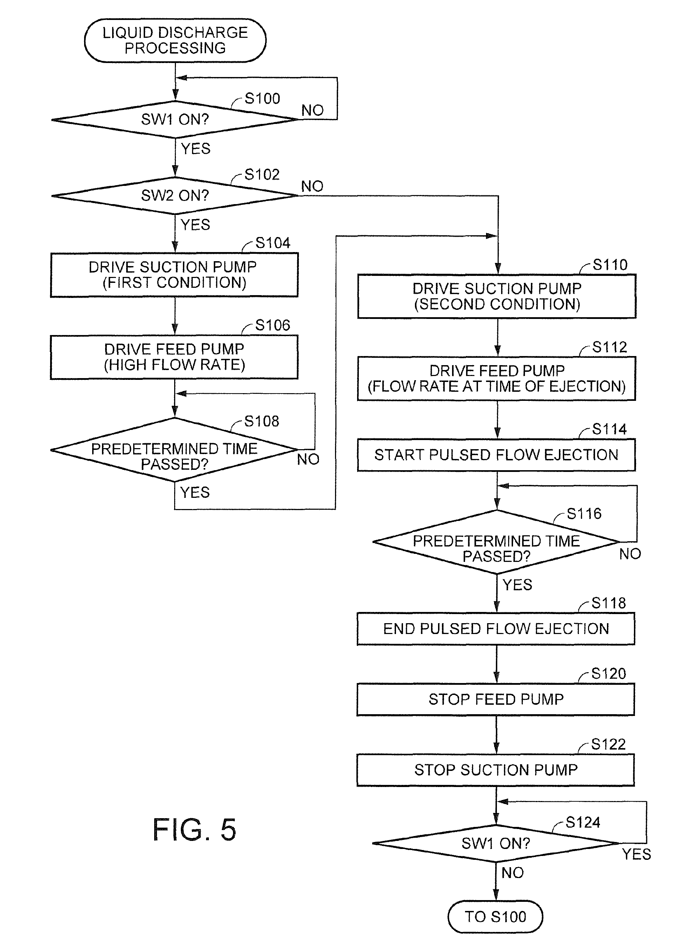

FIG. 5 is a flowchart of the liquid discharge processing executed by the control unit 400. This processing starts as the operator of the liquid ejection device system 10 starts up the liquid ejection device system 10. The processing is executed until the operation of the liquid ejection device system 10 is ended. As the liquid discharge processing is started, first, it is determined whether the first switch SW1 is ON or not (Step S100).

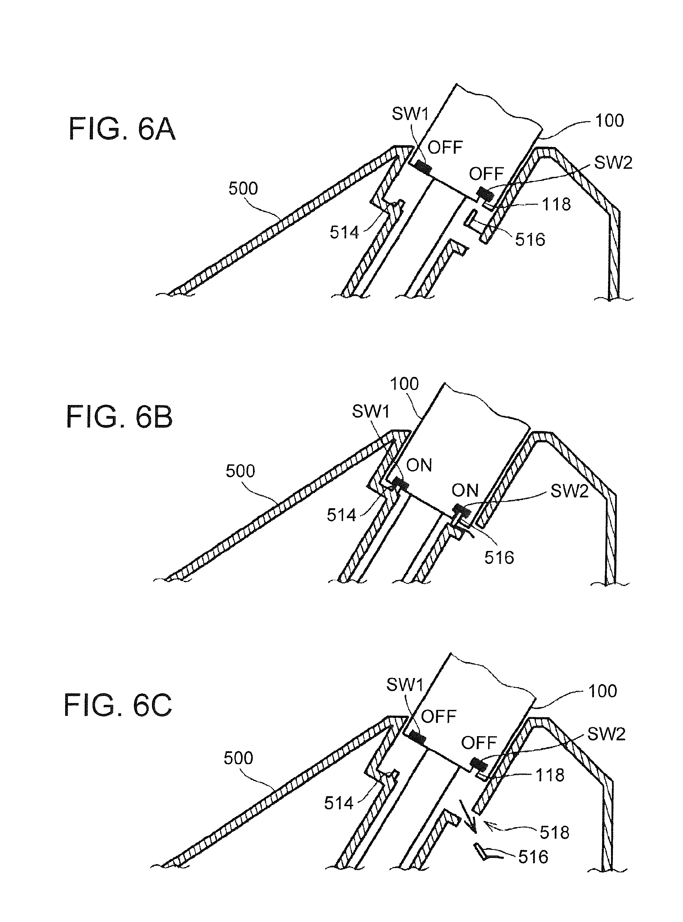

FIGS. 6A to 6C are explanatory views showing how the first and second switches SW1, SW2 are operated when the liquid ejection device 100 is set on the stand 500. In FIGS. 6A to 6C, the state when the liquid ejection device 100 is set on the stand 500 for the first time (hereinafter called a first round of setting) is shown.

As shown in FIG. 6A, in the housing passage 510 of the stand 500, the protrusion 514 is provided at the position corresponding to the first switch SW1 of the liquid ejection device 100, and the pin 516 is provided at the position corresponding to the second switch SW2 of the liquid ejection device 100. Therefore, as the liquid ejection device 100 is set on the stand 500, the first switch SW1 is pressed by the protrusion 514 and turns ON, and the second switch SW2 is pressed by the distal end of the pin 516 inserted in the communication path 118 of the liquid ejection device 100 and turns ON, as shown in FIG. 6B.

Here, once the liquid ejection device is set on the stand 500, the connection member connecting the pin 516 to the inner wall of the housing passage 510 is cut (see FIG. 6B). As the liquid ejection device 100 is detached from the stand 500 in this state, the pin 516 comes out of the communication path 118 and falls down from the penetrating portion 518, as shown in FIG. 6C. Consequently, when the liquid ejection device 100 is set on the stand 500 again (hereinafter called another round of setting), the second switch SW2 does not turn ON and only the first switch SW1 turns ON.

In this example, the structure in which the pin 516 in the housing passage 510 is eliminated once the liquid ejection device is set on the stand 500, so that the second switch SW2 does not turn ON in another round setting, is described. However, the structure of this example is not limiting, as long as both the first switch SW1 and the second switch SW2 turn on in the first round of setting, whereas the second switch SW2 does not turn ON and only the first switch SW1 turns on in another round of setting. Therefore, for example, a structure in which the pin 516 is pressed when the liquid ejection device 100 is set on the stand 500 and in which the pressed pin 516 does not return to the state before being pressed may be employed. Alternatively, a structure in which a form change occurs, such as closing of the communication path 118 on the side of the liquid ejection device 100, preventing the second switch SW2 from turning ON, may be employed. Moreover, the control unit 400 may memorize the first turning ON of the second switch SW2 that takes place after power is turned on, and may ignore the second turning ON and onward.

In this way, the first switch SW1 of the liquid ejection device 100 turns ON when the liquid ejection device 100 is set on the stand 500, irrespective of whether it is the first round of setting or another round of setting. Thus, in the liquid discharge processing of this example shown in FIG. 5, as the processing starts, whether the first switch SW1 is ON or not is determined first, thereby determining whether the liquid ejection device 100 is set on the stand 500 or not (Step S100). If it is determined that the first switch SW1 is ON (the liquid ejection device 100 is set on the stand 500) (Step S100: yes), then, whether the second switch SW2 of the liquid ejection device 100 is ON or OFF is determined (Step S102).

As described above, the second switch SW2 of the liquid ejection device 100 turns ON only in the first round of setting of the liquid ejection device 100 (see FIGS. 6A to 6C). Thus, in the liquid discharge processing of this example, if the first switch SW1 of the liquid ejection device 100 is ON and the second switch SW2 is ON (Step S100: yes, Step S102: yes), it is determined that this time is the first round of setting (the liquid chamber 112 of the liquid ejection device 100 is empty), and the initial filling operation of the liquid chamber 112 is carried out.

In the case of carrying out the initial filling operation, first, the suction pump 300 is driven under a first condition (amount of suction: large) for initial filling (Step S104), and the feed pump 200 is driven in this state (Step S106). The driving of the feed pump 200 is carried out in such a way as to supply the liquid to the liquid chamber 112 at a high flow rate (in this example, 30 ml per minute) that is equal to or higher than the flow rate used for excision or incision of a living tissue. In this case, the liquid does not overflow from the stand 500, because the liquid storage section 520 has an opening only at the inserting position of the liquid ejection device and the liquid flowing out to the liquid storage section 520 is sucked out from the suction opening 134. Thus, the supply of the liquid is continued until a predetermined first time passes after the liquid begins to be supplied to the liquid chamber 112 (Step S108: no). Then, as the predetermined first time passes (Step S108: yes), it is determined that the initial filling operation is complete. The processing subsequently shifts to the processing of the liquid discharge operation (Step S110 and onward).

The liquid discharge operation is carried out by ejecting the liquid in a pulse form from the nozzle 124 for a predetermined second time (pulsed flow ejection). Therefore, the drive condition of the suction pump 300 is changed to a second condition (amount of suction: medium) for liquid discharge (Step S110) and the flow rate of the liquid by the feed pump 200 is changed to a flow rate for ejection (in this example, 10 ml per minute) (Step S112). Then, as a drive voltage is applied to the piezoelectric element 116, pulsed flow ejection is started (Step S114). Since the suction pump 300 is also driven while the pulsed flow ejection is carried out, the liquid ejected to the liquid storage section 520 is sucked out from the suction opening 134.

In the initial filling operation, a high flow rate of the feed pump 200 is used in order to reduce the preparation period before the use of the liquid ejection device 100 is started. However, in the liquid discharge operation, the flow rate of the feed pump 200 is lower than in the initial filling. Therefore, the possibility of occurrence of a circumstance where the flow speed of the fluid inside the liquid ejection device 100 is too fast, causing air bubbles to be fixed at the end part or the like inside the liquid ejection device 100 and not to be discharged from the liquid ejection device 100, can be restrained. By lowering the flow rate of the feed pump 200, discharge of air bubbles at the end part or the like inside the liquid ejection device 100 is facilitated.

After the pulsed flow ejection is thus started, the pulsed flow ejection is continued until a predetermined time passes (Step S116: no). As the predetermined time passes (Step S116: yes), the pulsed flow ejection is ended (Step S118) and the feed pump 200 and the suction pump 300 are stopped (Steps S120, S122). The liquid discharge operation to discharge air bubbles is thus ended.

As the liquid discharge operation is ended, whether the first switch SW1 of the liquid ejection device 100 is ON or not is determined (Step S124). If the liquid ejection device 100 is left set on the stand 500, it is determined that the first switch SW1 is ON (Step S124: yes) and the device enters a standby state. Then, as the liquid ejection device 100 is detached from the stand 500 and the first switch SW1 turns OFF (Step S124: no), the processing flow returns to the beginning of the liquid discharge processing, and whether the liquid ejection device 100 is set on the stand 500 again or not is determined (Step S100).

As the liquid ejection device 100 is set on the stand 500 again, the first switch SW1 turns ON (Step S100: yes). However, the second switch SW2 remains OFF (Step S102: no) because the pin 516 for turning the second switch SW2 ON is already eliminated. In this case, the suction pump 300 is driven under the second condition for liquid discharge to discharge air bubbles (Step S110) and the feed pump 200 is driven, without carrying out the initial filling operation (Steps S104, S106, S108). Then, while the liquid is supplied to the liquid chamber 112 (Step S112), pulsed flow ejection is started (S114), thus carrying out the liquid discharge operation to discharge air bubbles. As a predetermined time passes after the pulsed flow ejection is started (Step S116: yes), the pulsed flow ejection is ended (Step S118) and the feed pump 200 and the suction pump 300 are stopped (Steps S120, S122). In this state, a standby state is kept until the liquid ejection device 100 is detached from the stand 500 (until the first switch SW1 turns OFF). Then, as the first switch SW1 of the liquid ejection device 100 turns OFF (Step S124: no), the processing flow returns to the beginning of the liquid discharge processing. The similar processing is repeated until the operation of the liquid ejection device system 10 ends.

In this way, in the stand 500 of this embodiment, simply setting the liquid ejection device 100 can cause the liquid ejection device system 10 to automatically carry out the initial filling operation and the liquid discharge operation to discharge air bubbles. Therefore, if the operator uses the stand 500, the operator need not to carry out these operations manually and therefore the burden on the operator can be reduced. Also, if the liquid ejection device 100 is returned on the stand 500 during a surgical operation or the like, the liquid discharge operation to discharge air bubbles is carried out during that period. Therefore, when resuming the operation of the liquid ejection device 100, the operation can be resumed in a good condition (state where air bubbles in the liquid chamber are discharged).

The liquid that flows out to the liquid storage section 520 of the stand 500 from the nozzle 124 when the initial filling operation and the liquid discharge operation to discharge air bubbles are carried out is sucked out by the suction pump 300 connected to the liquid ejection device 100. Thus, a passage or the like for discharging outside the liquid accumulated in the liquid storage section 520 need not be provided in the stand 500 and therefore the structure of the stand 500 can be simplified. Moreover, as described above with reference to FIG. 2, in the state where the liquid ejection device 100 is set on the stand 500, the gap between the outer periphery of the suction tube 130 and the housing passage 510 of the stand 500 is closed by the seal portion 512, thus sealing the liquid storage section 520. If the initial filling operation and the liquid discharge operation to discharge air bubbles are carried out in this state, the pressure inside the liquid storage section 520 becomes negative due to the suction force of the suction pump 300, and this negative pressure causes the liquid to be sucked into the liquid storage section 520 from the nozzle 124. Therefore, it is possible to carry out the initial filling operation and the liquid discharge operation to discharge air bubbles, using not only the force with which the liquid ejection device 100 ejects the liquid and the force with which the feed pump feeds the liquid under pressure, but also the suction force of the suction pump 300.

In addition, in the stand 500 of this embodiment, the initial filling operation of the liquid chamber 112 is carried out only when the liquid ejection device 100 is set for the first time. Therefore, when carrying out the initial filling operation (when starting the use of the liquid ejection device 100), an unused stand 500 needs to be prepared. Therefore, the use of the liquid ejection device 100 set on a used stand 500 can be restrained and the liquid ejection device 100 can be kept hygienic. Thus, in view of infection prevention, it is preferable to use the stand 500 of this embodiment as a medical apparatus.

### 2 Second Embodiment

Hereinafter, a second embodiment of the invention will be described. The description focuses on different parts from the liquid ejection device system 10 of the first embodiment. Similar configurations to those of the first embodiment are denoted by the same reference numerals and will not be described further in detail.

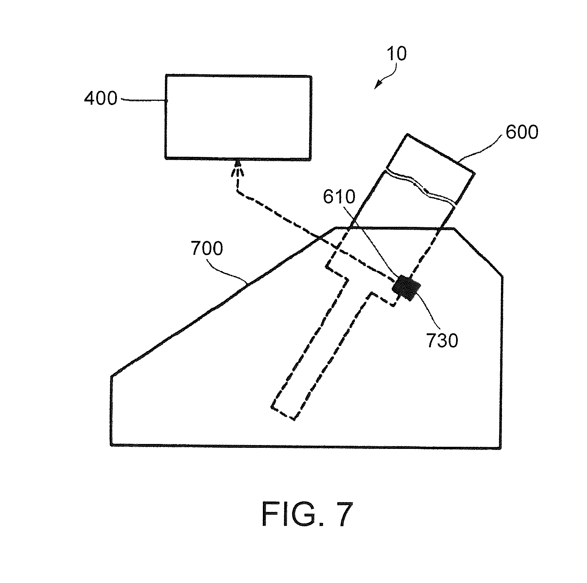

FIG. 7 is an explanatory view showing the state where a liquid ejection device 600 is installed on a stand 700 as the second embodiment. The illustrated stand 700 is provided with an ID display portion 730 to which an identification ID that is unique to each stand 700 is attached. In the liquid ejection device 600, a reading unit 610 which reads the identification ID is provided at the position corresponding to the ID display portion 730 of the stand 700 when the liquid ejection device 600 is set on the stand 700. Data read by the reading unit 610 is transmitted to the control unit 400 via the cable CBL.

In the stand 700 of the second embodiment, the identification ID read by the reading unit 610 can be outputted to the control unit 400, thus causing the control unit 400 to determine whether this identification ID is the same as an identification ID that is read in the past or not. Also with this configuration, whether the liquid ejection device 600 is set on the stand 700 for the first time or not can be determined. Therefore, as with the stand 500 of the first embodiment, the content of the liquid discharge processing executed in the liquid ejection device 600 can be changed between when the liquid ejection device 600 is set on the stand 700 for the first time and when the liquid ejection device 600 is set thereon for the second time and onward.

According to this configuration, whether the liquid ejection device 600 is installed on the stand 700 for the first time or the second time and onward can be detected without providing a movable part. Therefore, the operation is more secure.

### 3 Third Embodiment

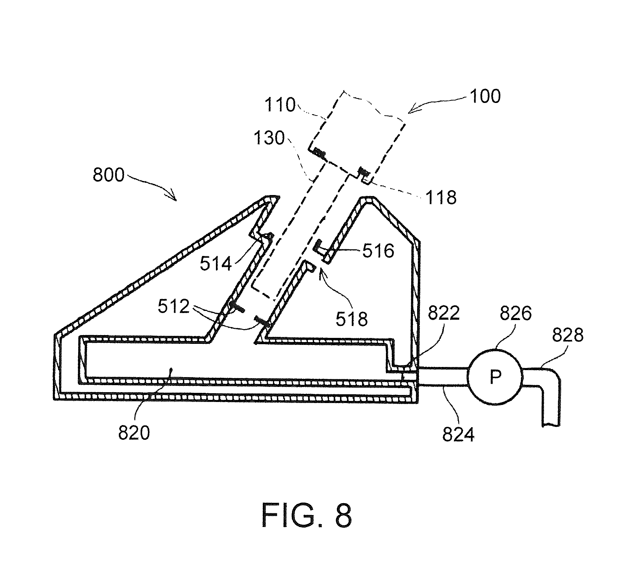

Next, a third embodiment of the invention will be described. FIG. 8 shows the schematic configuration of a stand 800 as the third embodiment. The stand 800 of the third embodiment has a liquid storage section 820, as illustrated. This liquid storage section 820 is connected to a stand-side suction pump 826 via a discharge passage 822 and a first drainage tube 824. The stand-side suction pump 826 is connected to the drainage container 320 (see FIG. 2) in which the liquid sucked by the suction pump 300 on the side of the liquid ejection device 100 is housed, via a second drainage tube 828.

As the stand-side suction pump 826 is thus provided for the stand 800, the liquid that flows out to the liquid storage section 820 in the initial filling operation and the liquid discharge operation to discharge air bubbles can be sucked using the strand-side pump 826. Also, if the stand-side suction pump 826 is driven in the state where the liquid ejection device 100 is inserted in the stand 800, a higher negative pressure than the negative pressure generated by the suction pump 300 connected of the liquid ejection device 100 can be generated to suck the liquid out of the nozzle 124. Therefore, even if the nozzle 124 is clogged, the clogging can be solved.

While the drainage sucked out by the stand-side suction pump 826 is discharged to the drainage container 320 in this embodiment, the drainage may be discharged to another separate container. The stand-side suction pump 826 may be driven in response to a signal from the control unit 400. Alternatively, a switch or sensor that directly detects that the liquid ejection device 100 is installed may be provided in the stand 800, and the stand-side suction pump 826 may be directly driven by using an output from the switch or the like. Also, a manually operable switch may be provided in the stand 800. As the switch is operated in the state where the liquid ejection device 100 is installed on the stand 800, the stand-side suction pump 826 may be driven to generate a high negative pressure, thus solving the clogging.

### Modification

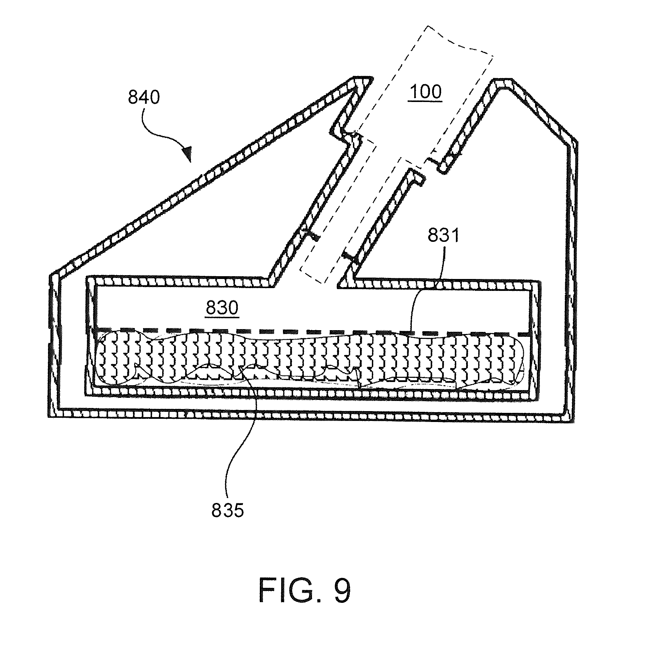

In the embodiment, the liquid accumulated in the liquid storage section 820 is sucked by the stand-side suction pump 826 and discharged outside. However, a configuration in which the liquid is not discharged outside may also be employed. For example, as shown in FIG. 9, a sodium polyacrylate-based superabsorbent polymer 835 may be arranged in a liquid storage section 830 and the liquid that leaks out or is ejected from the liquid ejection device 100 may be adsorbed by the superabsorbent polymer 835. The superabsorbent polymer 835 is not limited to the sodium polyacrylate-based superabsorbent polymer and any highly absorbent polymer may be used. A resin polymerized with a water-soluble monomer such as polyvinyl alcohol or polyethylene glycol, or the like may be used.

According to this modification, the liquid discharged from the liquid ejection device 100 may be contained inside a stand 840 and disposed of with the stand 840 after the surgical operation ends. This provides high levels of hygiene. Also, since the drainage is adsorbed by the superabsorbent polymer 835, the drainage does not contact the liquid ejection device 100 even if the stand 840 becomes inclined or falls down. Again, this provides high levels of hygiene. In order to prevent the superabsorbent polymer 835 from moving or fluctuating in the liquid storage section 830, it is effective to hold the superabsorbent polymer 835 with a net or mesh 831.

### 4 Fourth Embodiment

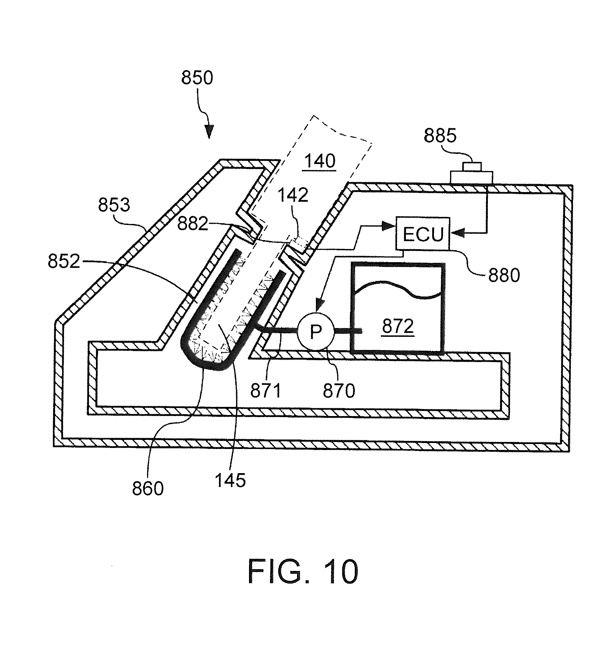

Next, a fourth embodiment of the invention will be described. FIG. 10 is an explanatory view showing the internal structure of a stand 850 of the fourth embodiment, as viewed in a cross-sectional view. This stand 850 has, in a casing 853, a housing passage 852 for housing a liquid ejection device 140, as in the first embodiment or the like. The stand 850 of this embodiment has a cleaning nozzle 860 in the housing passage 852. The cleaning nozzle 860 is arranged to surround the suction tube of the liquid ejection device 140 housed in the housing passage 852. A cleaning pump 870 is connected to the cleaning nozzle 860 via a passage 871. The cleaning pump 870 can pump out a cleaning solution from a cleaning solution tank 872 and feed the cleaning solution toward the cleaning nozzle 860.

The cleaning pump 870 is ON/OFF-controlled by an electronic control unit (ECU) 880 housed in the stand 850. The ECU 880 and the cleaning pump 870 operate on receiving power supplies, not shown. These power supplies may be disposable power supplies housed in the stand 850 or may be provided from outside. The stand 850 is further provided with a sensor 882 which detects the installation of the liquid ejection device 140 and with a manual switch 885. Of these, the sensor 882 is a Hall element that operates with a magnet 142 provided in the liquid ejection device 140. As the liquid ejection device 140 is inserted in the housing passage 852, the sensor 882 turns ON due to the magnetic force of the magnet 142. Both the sensor 882 and the manual switch 885 are connected to the ECU 880. The ECU 880 includes a CPU, flash ROM, RAM or the like, not shown, and repeatedly executes a program that is written in the ROM in advance, thus realizing the following operation.

As the use of the stand 850 is started and the liquid ejection device 140 is installed in the housing passage 852 for the first time, the sensor 882 turns ON. The ECU 880 then detects that the sensor 882 is ON, drives the cleaning pump 870 to eject the cleaning solution from the cleaning nozzle 860 for a short time, and thus cleans a suction tube 145 or the like of the liquid ejection device 140. This operation ends in a few seconds. After that, the liquid ejection device 140 is taken out of the stand 850. Subsequently, every time the liquid ejection device 140 is returned into the stand 850, the cleaning pump 870 is similarly driven to carry out cleaning for a short time.

Meanwhile, if the user operates (turns ON) the manual switch 885 in the state where the liquid ejection device 140 is installed in the stand 850, the ECU 880 detects this operation and drives the cleaning pump 870 to eject cleaning solution from the cleaning nozzle 860 while the manual switch 885 is ON, thus cleaning the liquid ejection device 140.

According to the fourth embodiment, the distal end and the suction tube 145 of the liquid ejection device 140 can be cleaned every time the liquid ejection device 140 is installed in the stand 850. Thus, the part at the distal end including the ejection nozzle of the liquid ejection device 140 can be kept clean. The cleaning operation can also be carried out manually. When there are particularly bad stains, the manual switch 885 can be operated to clean the liquid ejection device 140. The cleaning solution used for the cleaning may be discharged outside by another pump, as in the third embodiment, or may be adsorbed by a superabsorbent polymer, as in the modification of the third embodiment. The cleaning pump 870 and the cleaning solution tank 872 may be prepared inside the stand 850 in advance, or may be prepared outside and connected with a control signal line so that the cleaning pump 870 and the cleaning solution tank 872 can be controlled by the ECU 880 in the stand 850.

### 5 Fifth Embodiment

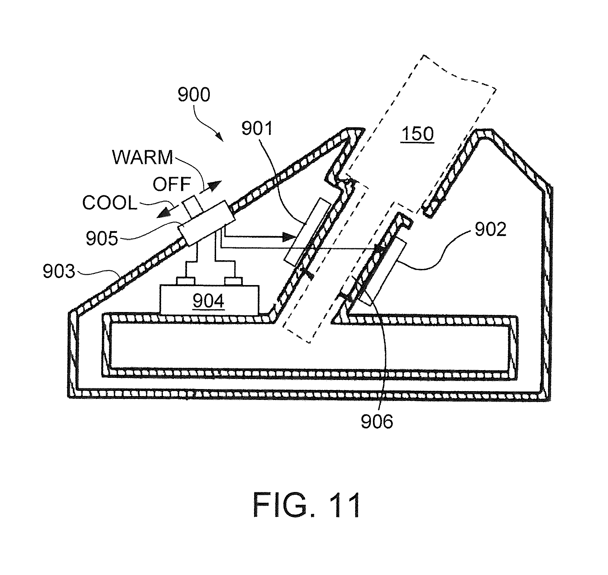

Next, a fifth embodiment of the invention will be described with reference to Fig. 11. A stand 900 of the fifth embodiment has a substantially similar shape to the stand 500 of the first embodiment. The stand 900 of the fifth embodiment has Peltier elements 901, 902 on the outside of a housing passage 906 for housing a liquid ejection device 150. The Peltier elements 901, 902 are connected to a DC power source 904 provided within the stand 900, via a three-position slide switch 905 provided in a housing 903.

The Peltier elements 901, 902 are provided with electrodes on both sides thereof, and transfers heat from inside (housing passage side) to outside or in the opposite direction, depending on the direction of a voltage applied between the electrodes. Positive and negative electrodes of the DC power source 904 are connected to two common terminals (c-terminals) of the slide switch 905, and α-terminal and b-terminal are connected to the positive and negative electrodes of each of the Peltier elements 901, 902 in a crossover manner. Therefore, when the three-position slide switch 905 is at the center position (OFF position), the common terminals are connected to neither one of the α-terminal and the b-terminal, and no voltage is applied to the Peltier elements 901, 902. Therefore, the Peltier elements 901, 902 do not transfer heat at this point.

If a knob on the slide switch 905 is moved to the position of "Cool", electrical connection is formed between the common terminal and the a-terminal, and a voltage from the DC power source 904 is applied between the respective electrodes of the Peltier elements 901, 902 so that heat is transferred from inside to outside. Consequently, the inner side of the Peltier elements 901, 902, that is, the side of the housing passage 906 is cooled. If the liquid ejection device 150 is housed in the housing passage 906, the suction tube, the ejection nozzle and the like are cooled.

Meanwhile, if the knob on the slide switch 905 is moved to the position of "Warm" that is opposite to "Cool", electrical connection is formed between the common terminal and the b-terminal, and a voltage from the DC power source 904 is applied between the respective electrodes of the Peltier elements 901, 902 so that heat is transferred from outside to inside. Consequently, temperature rises on the inner side of the Peltier elements 901, 902, that is, on the side of the housing passage 906. If the liquid ejection device 150 is housed in the housing passage 906, the suction tube, the ejection nozzle and the like are heated.

According to the fifth embodiment, the liquid ejection device 150 housed in the stand 900 can be cooled and heated simply by operating the slide switch 905. Therefore, the liquid ejection device 150 can be operated in the state where the temperature thereof is adapted to the state of the patient subjected to the surgical operation or the form of the surgical operation. For example, in a surgical operation where the patient is maintained in a hypothermic state, cooling the liquid ejection device 150 prevents the hypothermic state from being disturbed. Also, while the DC power source 904 is provided within the stand 900 in this embodiment, the power source may also be provided from outside. Thermal pumps may be used instead of the Peltier elements. A configuration in which only cooling or only heating is carried out may also be employed. In the case of cooling only, a refrigerant may be housed inside the stand 900 and the stand 900 may be stored in a refrigerator for a predetermined time before the surgical operation. In the case of heating only, a heater may be used, or a technique of generating heat by adding water to calcium hydroxide may be used. High-frequency heating or the like may also be used.

### 6 Sixth Embodiment

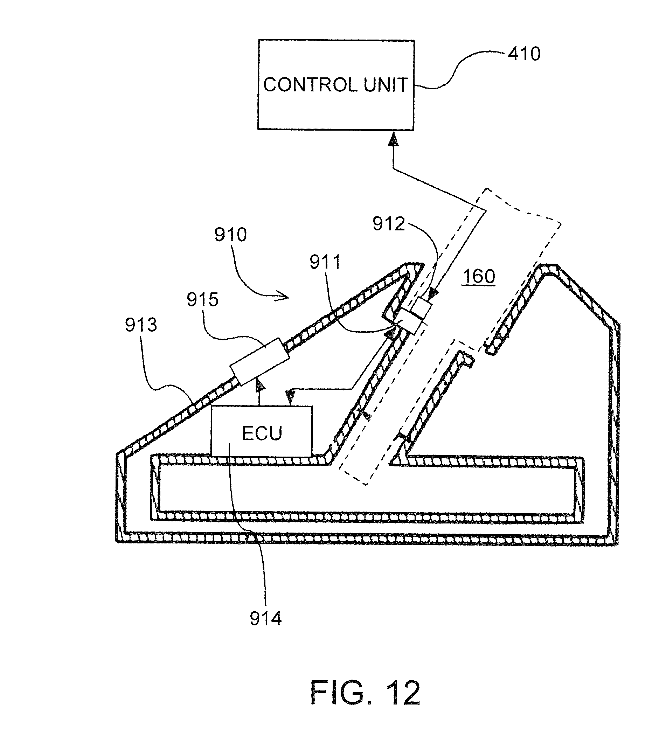

A stand 910 according to a sixth embodiment has an ECU 914 inside a casing 913, as shown in FIG. 12. A communication coupler 911 for communication and a display unit 915 are connected to the ECU 914. The ECU 914 includes a CPU, flash ROM, RAM and the like, not shown, as in the fourth embodiment. The ECU 914 is connected to the communication coupler 911 via a serial communication port. The communication coupler 911 is provided in a housing passage of the stand 910, and as a liquid ejection device 160 is housed therein, the communication coupler 911 is enabled to communicate with a communication coupler 912 on the side of the liquid ejection device 160. The communication between the two communication couplers 911 and 912 may be optical communication or may use electromagnetic induction or the like. Of course, a configuration in which plural terminals directly electrically contact each other to enable communication may be employed. The communication coupler 912 on the side of the liquid ejection device 160 is connected to a control unit 410. Therefore, the ECU 914 in the stand 910 can communicate with the control unit 410 having similar functions to the control unit 400 described in the first embodiment.

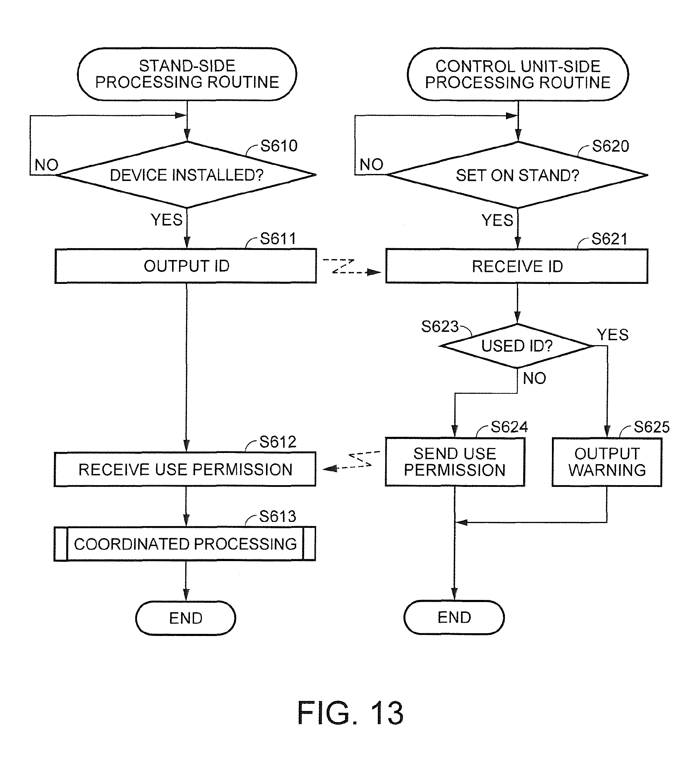

Thus, the communication of data between the ECU 914 and the control unit 410 will be described with reference to FIG. 13. The left side of FIG. 13 shows a stand-side processing routine carried out by the ECU 914 in the stand 910, whereas the right side shows a control unit-side processing routine carried out on the side of the control unit 410. As the use of the stand 910 is started, the ECU 914 first repeatedly detects whether the liquid ejection device 160 is installed in the housing passage or not (Step S610). Meanwhile, as power is turned on and the liquid ejection device 160 is connected, the control unit 410 carries out an initial operation to confirm whether the liquid ejection device 160 is a normal device or not, and then repeatedly detects whether the liquid ejection device 160 is set on the stand or not (Step S620). In this embodiment, whether the liquid ejection device 160 is set on the stand and installed in the housing passage or not is detected in accordance with whether the ECU 914 and the control unit 410 are enabled to communicate via the communication couplers 911 and 912. Of course, sensors such as Hall elements may be provided to detect each other.

As the liquid ejection device 160 is installed in the housing passage and communication using the communication couplers is enabled, the ECU 914 carries out processing to output an ID allocated to the stand (Step S611). The ID is an identifier that can identify each stand 910, and is written in the flash ROM provided inside the ECU 914. The ID outputted via the communication coupler 911 is received by the control unit 410 (Step S621). As the control unit 410 receives the ID, the control unit 410 determines whether the received ID is a used ID or not (Step S623). Whether the ID is a used ID or not is determined by referring to a list of IDs recorded by the control unit 410 (list of IDs of stands used in the past) . If there is the same ID in the recorded list, it means that the stand is used in the past.

If the control unit 410 determines that the received stand ID is not a used ID (Step S623, "NO"), a notification of use permission is sent to the side of the stand 910 (Step S624). Meanwhile, if the control unit 410 determines that the received ID is a used ID (Step S623, "YES"), the control unit 410 outputs a warning (Step S625). The output of a warning can be carried out, for example, by displaying or outputting an audio saying "You're using a used stand. Stop using the stand immediately and replace the stand and handpiece with new ones", from the control unit 410. In addition to the warning, all the functions of the control unit 410 or the functions related to the use of the liquid ejection device 160 may be made unavailable for use.

As the use permission is sent from the side of the control unit 410 (Step S624), the ECU 914 in the stand 910 receives the use permission (Step S612) and executes coordinated processing (step S613). The coordinated processing is processing carried out on the assumption that the stand 910 and the liquid ejection device 160 housed in the housing passage thereof can be normally used. For example, the initial filling operation and the liquid discharge operation to discharge air bubbles described in the first embodiment are equivalent to the coordinated processing. Also, the suction processing of the ejection nozzle described in the third embodiment, the cleaning operation described in the fourth embodiment, the cooling or heating processing described in the fifth embodiment, and the like can also be carried out. When executing the coordinated processing, the ECU 914 may display the content of the processing on the display unit 915. The display unit 915 may also output the display content in the form of an audio output.

In the stand 910 according to the sixth embodiment, when the liquid ejection device 160 is installed, the stand 910 and the control unit 410 are enabled to communicate with each other, and data is exchanged and processed between the stand 910 and the control unit 410. Therefore, in addition to similar effects to the first and second embodiments, the ID can be exchanged between the stand 910 and the control unit 410 so that the use of a used stand can be prohibited more securely. Also, an excellent effect that the stand 910 and the control unit 410 can carry out coordinated processing is achieved.

### Modification

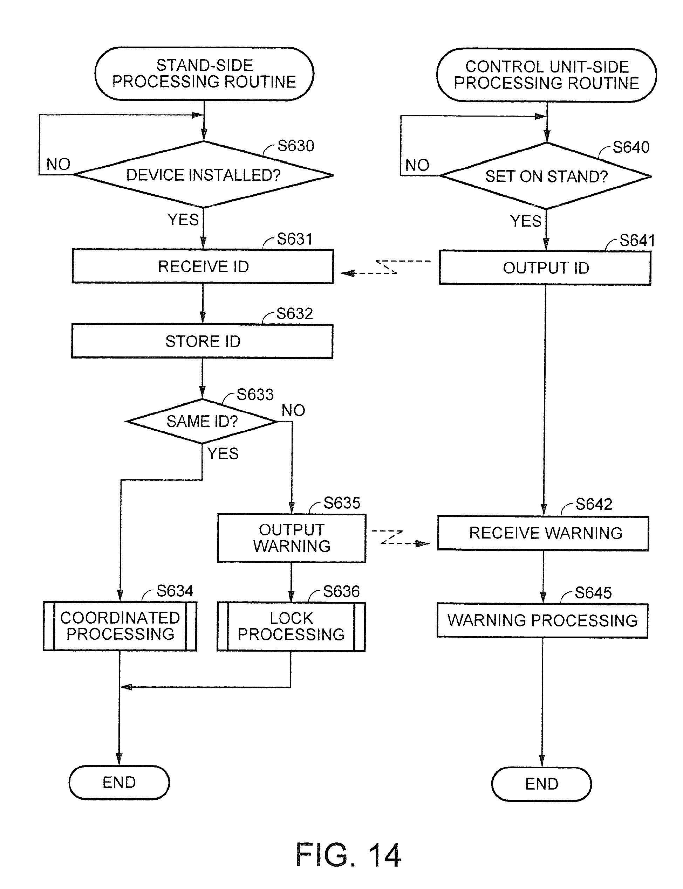

A modification of the embodiment will be described. FIG. 14 is a flowchart showing a stand-side processing routine and a control unit-side processing routine according to the modification. In the sixth embodiment, the ID is sent from the side of the stand 910, whereas in this modification, the ID is outputted from the side of the control unit 410. That is, the ECU 914 on the stand side checks the installation of the liquid ejection device 160 (Step S630) and the control unit 410 checks the installation of the liquid ejection device 160 on the stand (Step S640). Then, as the liquid ejection device 160 is installed on the stand 910 and communication between the two units is enabled, the ID of the stand is outputted from the side of the control unit 410 (Step S641). As power is turned on, the control unit 410 generates a new ID based on the time when power is turned on, and outputs the generated ID. Therefore, the ID is newly generated every time the control unit 410 is used once, that is, every single surgical operation.

The ECU on the side of the stand 910 receives the ID via the communication coupler 911 (Step S631) and stores this ID in the flash ROM in a non-volatile manner (Step S632). Subsequently, the ECU 914 on the side of the stand 910 determines whether the same ID is already stored in the flash ROM or not (Step S633). If the ECU 914 in the stand 910 receives an ID from outside, the ECU 914 sequentially stores the ID in a predetermined area. Next, the ECU 914 determines whether the ID that is received this time is the same as the ID that is received previously or not (Step S633). The ID outputted from the control unit 410 is the same for each single surgical operation. Therefore, if it is the same ID, coordinated processing is executed (Step S634), as in the sixth embodiment.

Meanwhile, if it is not the same ID, it means that a new liquid ejection device 160 is connected to the stand 910 that is already used once. Therefore, the ECU 914 outputs a warning (Step S635) and also carries out lock processing (Step S636). The lock processing is processing in which a solenoid, not shown, is operated to lock the liquid ejection device 160 so that the liquid ejection device 160 cannot be taken out of the stand 910. Thus, if a new liquid ejection device 160 is installed by mistake on the stand 910 that is used for another patient, the liquid ejection device 160 is prevented from being used.

As the stand 910 outputs a warning (Step S635), the control unit 410 receives the warning via the communication couplers 911 and 912 (Step S642). The control unit 410 then executes warning processing (Step S645). The warning processing is processing to enable the user to recognize an audio or display-based warning saying "Stop using the device" or the like, as in the sixth embodiment. Also, at least a part of the functions of the control unit may be stopped to make the liquid ejection device 160 unavailable for use.

According to this modification, as in the sixth embodiment, the situation where the stand 910 that is used once is used again for the surgical operation of another patient can be securely avoided. Moreover, in this modification, since the ID is generated on the side of the control unit 410, different IDs need not be provided one by one on the side of the stand 910. That is, a large number of stands with totally the same configuration can be produced in advance.

### 7 Seventh Embodiment

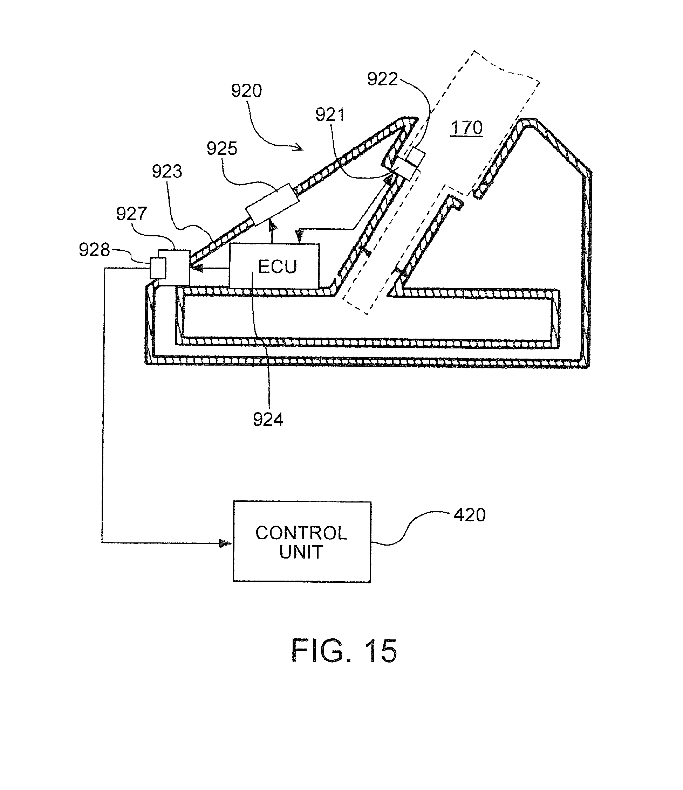

Next, a seventh embodiment of the invention will be described with reference to FIG. 15. A stand 920 of the seventh embodiment has a similar configuration to the sixth embodiment in that an ECU 924 is provided inside a casing 923 and that a display unit 925 or the like is provided on the surface of the casing 923. The stand 920 also has a sensor 921 which detects whether a liquid ejection device 170 is installed or not. The sensor 921 uses a Hall element and turns ON with a magnet 922 on the side of the liquid ejection device 170. This sensor 921 is connected to the ECU 924. Therefore, the ECU 924 can detect that the liquid ejection device 170 is installed on the stand 920.

The ECU 924 is also connected, via a signal line, to a communication connector 927 attached to the casing 923. A signal line from an external device can be connected to the communication connector 927 via a connector 928. In this embodiment a control unit 420 forming a liquid ejection device system is connected.

In the seventh embodiment having such a configuration, the stand 920 can detect the installation of the liquid ejection device 170 and output the detection directly to the external device, in this example, the control unit 420. Of course, the stand 920 can communicate not only the installation of the liquid ejection device 170 but also other control signals and data or the like with the control unit 420. Consequently, the stand 920 can execute coordinated processing with the control unit 420. For example, if the control unit 420 is connected to the feed pump 200 and the suction pump 300, as shown in FIG. 1, whether the liquid ejection device 170 is installed or not can be detected and these pumps can be controlled. Therefore, for example, when the liquid ejection device 170 installed on the stand 920 is detached from the stand 920, processing to detect this detachment and start up the feed pump 200 can be carried out. The liquid ejection device 170 detached from the stand 920 is immediately used for a surgical operation at a surgical site. In this case, the feed pump 200 is started up, triggered by the detachment of the liquid ejection device 170 from the stand 920. Therefore, by the time when the user moves the ejection nozzle at the distal end of the liquid ejection device 170 towards the surgical site, the liquid is already fed into the liquid ejection device 170 or at least about to reach the liquid ejection device 170. Consequently, the user can use the liquid ejection device 170 without having to wait for the liquid filling. Therefore, according to the seventh embodiment, the initial filling operation can be omitted.

Also, in the seventh embodiment, since the signal from the stand 920 is outputted directly to the control unit 420 without going through the liquid ejection device 170, it is possible to output, for example, information that the liquid ejection device 170 is not installed on the stand 920, to the control unit 420. Moreover, when installing the liquid ejection device 170, it is possible to output a large volume of data gathered by the ECU 924 from the liquid ejection device 170, directly to the control unit 420. Furthermore, the ECU 924 may communicate with another device than the control unit 420 or may communicate with another device as well as with the control unit 420. As another device, for example, a monitor device such as surgical operation monitor, a recording device, a voice recorder, a video recorder or the like can be used.

### 8 Eighth Embodiment

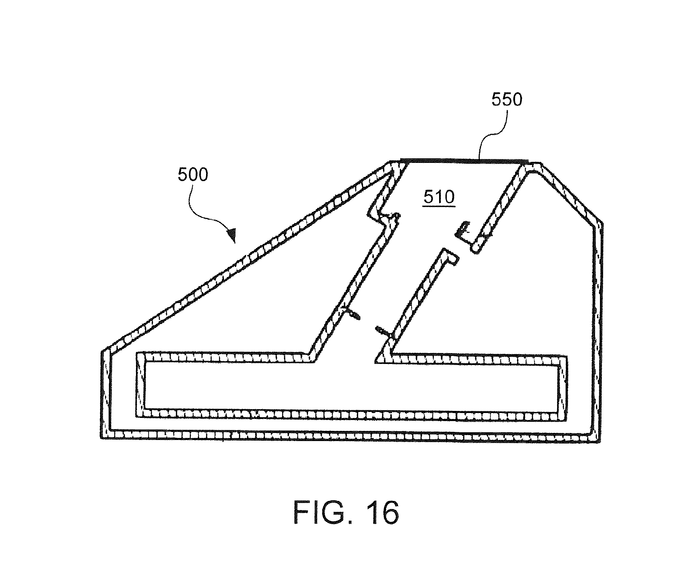

An eighth embodiment of the invention is shown in FIG. 16. A stand of the eighth embodiment has the same configuration as the stand 500 of the first embodiment and is different only in that a protection sheet 550 is provided at the inlet port of the housing passage 510. The protection sheet 550 is sterilized with the stand 500 and seals the inlet port of the housing passage 510. Therefore, there is no risk that foreign matters enter into the housing passage 510 before the installation of the liquid ejection device 100 after the stand 500 is set in an operation theater or the like.

The protection sheet 550 may manually stripped off immediately before the liquid ejection device 100 is housed in the housing passage 510, or may be made easily releasable by a thrust of the liquid ejection device 100. Alternatively, a water flow ejected from the ejection nozzle, which also serves for testing of the liquid ejection device 100, may be used to cut the protection sheet to open. Thus, the protection sheet 550 can be prevented from being opened before use, and the possibility of entry of foreign matters can be eliminated. The protection sheet 550 may be transparent to make the inside visible, or may be opaque. Also, as the protection sheet 550, an elastic sheet in an expanded state may be bonded to the inlet port of the housing passage. Thus, at the point when a part of the protection sheet 550 is cracked, the protection sheet 550 contracts due to its own tension and quickly opens the housing passage. Alternatively, in view of carrying out a surgical operation in a clean environment, it is preferable to form slits (for example, radial slits from the center) in the protection sheet 550 in advance so that, when the liquid ejection device 100 is inserted, the protection sheet 550 is divided along the slits and is less likely to produce broken pieces.

### 9 Ninth Embodiment

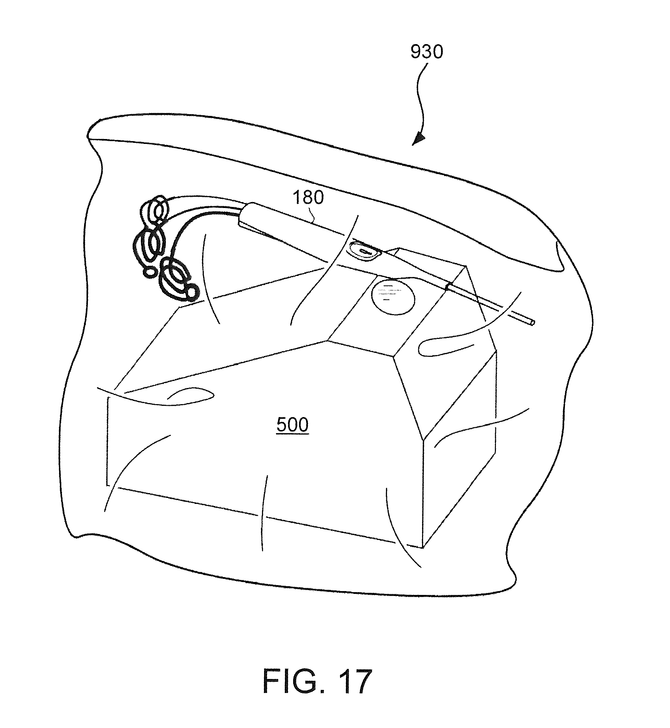

A ninth embodiment is shown in FIG. 17. As illustrated, this stand 500 is the stand of the first embodiment or the eighth embodiment. This stand is put in a storage bag 930 together with a handpiece 180 as a liquid ejection device and the bag is sealed. The stand 500 and the handpiece 180 in the state of being put in the storage bag 930 are sterilized with ultraviolet rays.

Since the stand 500 is put together with the handpiece 180 in the storage bag 930 and sterilized, by tearing the storage bag 930 to take out the stand 500 and the handpiece 180 in an operation theater or the like, the stand 500 and the handpiece 180 can be immediately used without having to sterilize these units. Moreover, it is clear that the stand 500 has never been used up to this point and there is no risk of the stand being used twice.

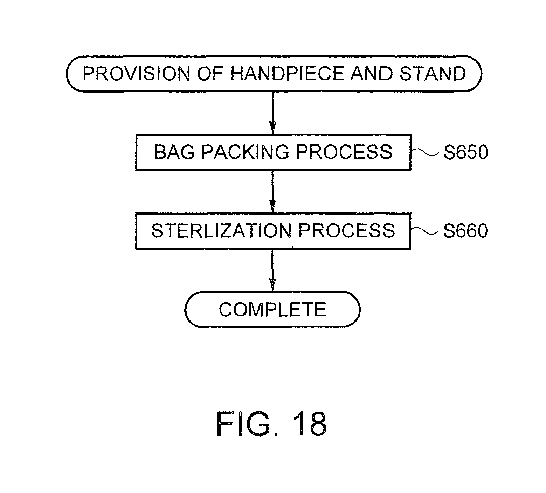

A method for providing the handpiece and the stand of the ninth embodiment is shown in FIG. 18. That is, the stand 500 and the handpiece 180 are packed in the bag (Process S650) and this is then sterilized with ultraviolet rays (Process S660). Thus, the stand 500 and the handpiece 180 that are sterilized and disinfected are provided in the state of being put in the storage bag 930. It is also possible to put only the stand 500 in the bag, and sterilize and provide the stand in the bag.

### 10 Tenth Embodiment

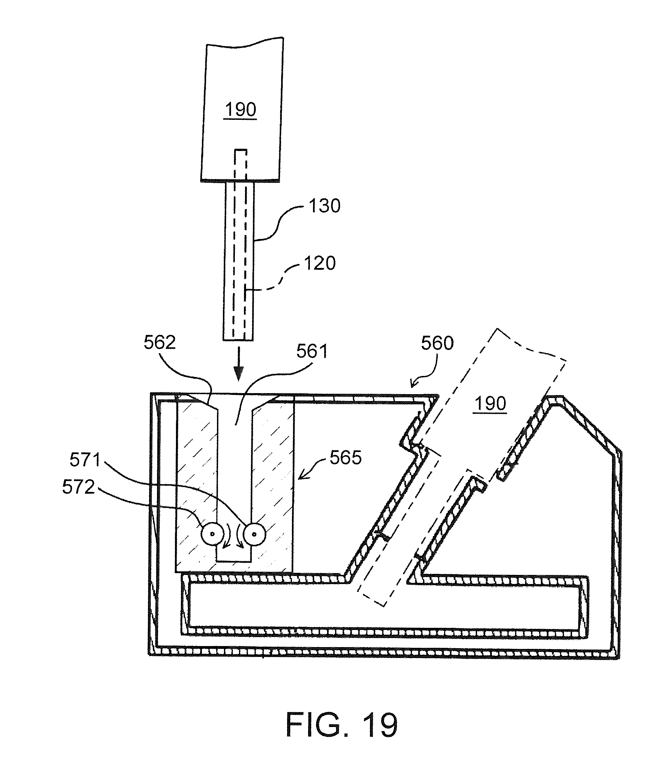

Next, a tenth embodiment of the invention will be described. FIG. 19 is an explanatory view showing the form of a stand 560 of the tenth embodiment, as viewed in a cross-sectional view. As illustrated, the stand 560 has a used suction tube storage unit 565, in addition to a housing passage for housing a liquid ejection device 190. The used suction tube storage unit 565 has an insertion portion 561 having a slightly larger inner diameter than the suction tube 130 of the liquid ejection device 190, an inclined portion 562 provided at the entrance of the insertion portion 561, and a pair of rollers 571, 572 provided near the bottom of the insertion portion 561, as illustrated. A simple one-way clutch is attached to the pair of rollers 571, 572 so that these rollers rotate only in one direction. The direction in which these rollers can rotate is counterclockwise as illustrated for the roller 571 and clockwise as illustrated for the roller 572. The separation distance between the outer circumferences of the pair of rollers 571, 572 is slightly smaller than the outer diameter of the suction tube 130.

While the liquid ejection device 190 is in use, the suction tube 130 carries out a suction operation to suck the liquid ejected to the surgical site, blood and the like. In this operation, the suction tube 130 may be clogged with tissues broken into fragments by droplets ejected from the ejection tube 120, making replacement of the suction tube 130 inevitable. In such a case, the user, still holding the liquid ejection device 190, may move the suction tube 130 toward the used suction tube storage unit 565 in the stand 560 from above and insert the suction tube 130 into the insertion portion 561. Since the inclined portion 562 is provided at the entrance of the insertion portion 561, the suction tube 130 is guided into the insertion portion 561 as the suction tube 130 is moved toward the insertion portion 561 from above.

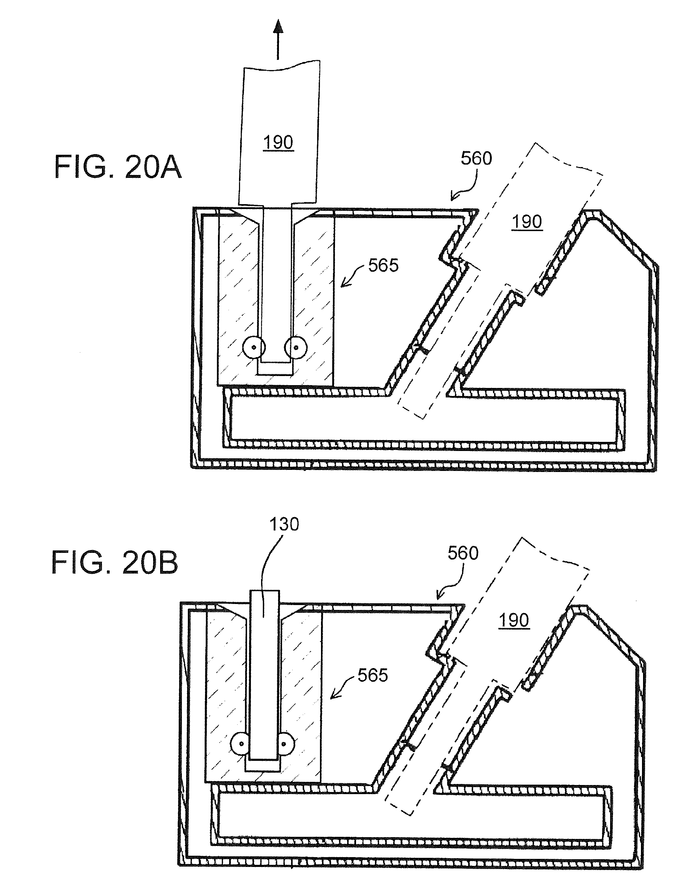

As the suction tube 130 is inserted further into the insertion portion 561, the distal end of the suction tube 130 eventually touches the pair of rollers 571, 572. Since the pair of rollers 571, 572 are rotatable in the direction in which the suction tube 130 is inserted, the rollers rotate in the way of holding the suction tube 130 from both sides, and the suction tube 130 is inserted further into the insertion portion 561. This state is shown in FIG. 20A.

Next, the liquid ejection device 190 is lifted upward (in the direction of the arrow) from the state shown in FIG. 20A. In this case, since the pair of rollers 571, 572 do not rotate backward, the suction tube 130 remains held between the pair of rollers 571, 572 and cannot move. Therefore, as the liquid ejection device 190 is lifted upward, the suction tube 130 is detached from the liquid ejection device 190 and remains in the used suction tube storage unit 565. This state is shown in FIG. 20B.

According to the stand 560 of the tenth embodiment, when the suction tube 130 needs to be replaced due to clogging or the like, the user only has to insert the suction tube 130 into the used suction tube storage unit 565 prepared in the stand 560 and then lift the liquid ejection device 190 up. Simply by this operation, the used suction tube 130 is detached from the liquid ejection device 190 and is held in the used suction tube storage unit 565. Therefore, the user need not carry out a manual operation to remove the suction tube 130. Thus, the situation where the user who is trying to remove the suction tube 130 ends up damaging the gloves by being caught by the metallic ejection nozzle or the like, is avoided. Cansequently, there is no risk that the surgical operator who is trying to remove the suction tube 130 may be infected from the patient's bodily fluids or the like.

### 11 Eleventh Embodiment

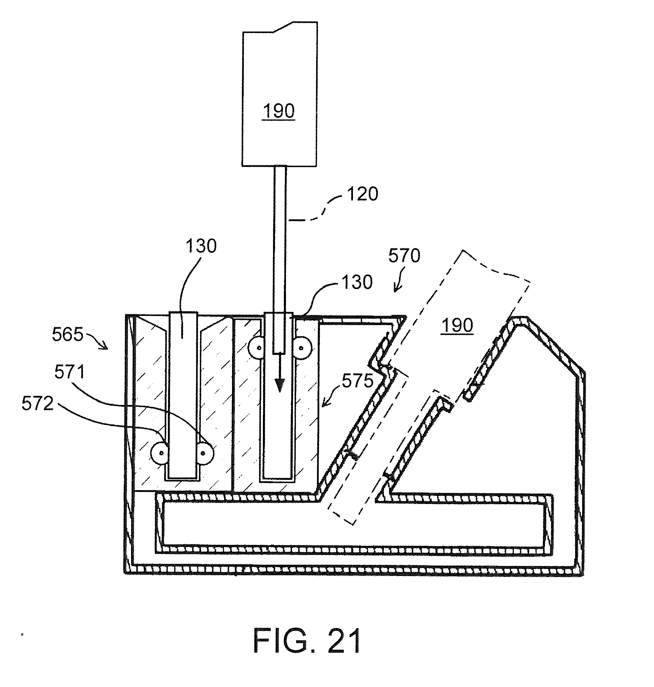

In the tenth embodiment, the configuration with the removable suction tube 130 is described. However, in an eleventh embodiment, a configuration in which the suction tube 130 is not only removable but also a new suction tube is attachable is described. The configuration of this stand 570 is shown in FIG. 21. As illustrated, a replacement suction tube storage unit 575 is provided next to the used suction tube storage unit 565. The replacement suction tube storage unit 575 has a very similar configuration to the used suction tube storage unit 565. However, these two units are different in that the used suction tube storage unit 565 has an empty space inside before use, whereas the replacement suction tube storage unit 575 holds an unused suction tube 130 inside. Also, a pair of rollers provided in the replacement suction tube storage unit 575 are rotatable in both directions and simply hold the new suction tube 130.

As described in the tenth embodiment, after the old suction tube 130 of the liquid ejection device 190 is inserted into the used suction tube storage unit 565 and the suction tube 130 is detached, the liquid ejection tub 120 of the liquid ejection device 190 is inserted into the new suction tube 130 in the replacement suction tube storage unit 575. Then, the liquid ejection tube 120 is inserted into the replacement suction tube storage unit 575 until the suction tube 130 is fitted with and attached to the main body of the liquid ej ection device 190.

By this operation, the suction tube 130 is replaced with a new one. Therefore, even if the suction tube 130 is clogged during a surgical operation, the detachment of the old suction tube 130 and the installation of the unused suction tube 130 can be easily carried out using the stand 570. Thus, the user of the liquid ejection device 190 need not hold the suction tube 130 or carry out the installation operation with the hands wearing gloves at the time of replacing the suction tube 130. Therefore, the risk of damage to the gloves, infection and the like can be avoided.

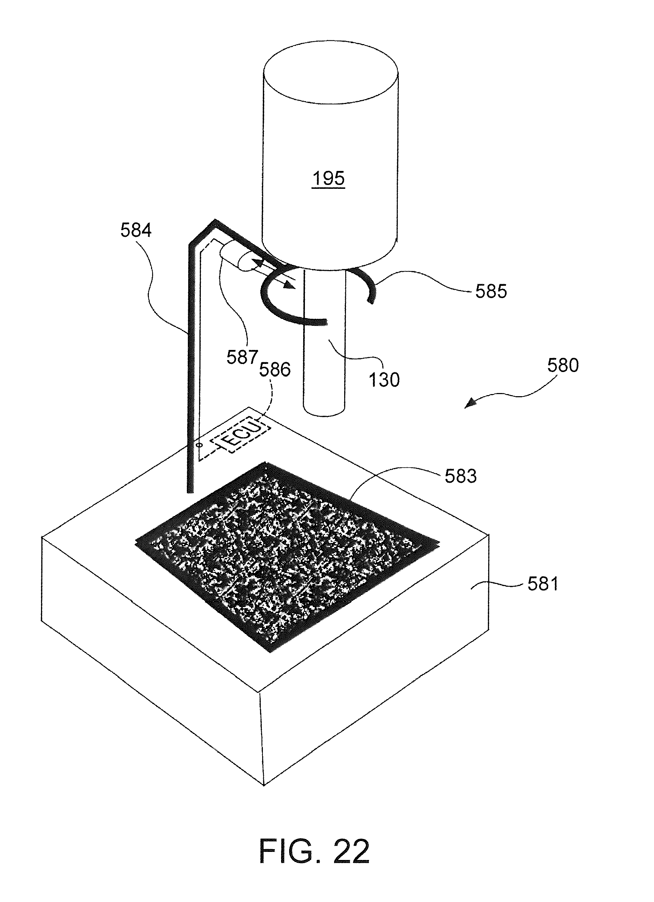

Several embodiments of the stand according to the invention are described above. However, the invention is not limited to the embodiments and can be carried out in various forms without departing from the scope of the invention. For example, the shape of the stand is not limited to the shapes of the first to eleventh embodiments. An open shape as illustrated in FIG. 22 may also be employed. In a stand 580 shown in FIG. 22, a liquid ejection device 195 is installed on a holder 584 provided upright from a stand main body 581. In this example, the liquid ejection device 195 is accommodated in a ring portion 585 provided at the distal end of the holder 584. In the example of FIG. 22, a part of the ring 585 (forward side) is cut out in order to prioritize easy entry of the liquid ejection device 195. However, a closed ring can also be used.

In the stand 580 shown in FIG. 22, a sensor 587 is provided on the holder 584. The sensor 587 detects whether the liquid ejection device 195 is accommodated or not, and outputs the result of the detection to an ECU 586 provided in the stand main body 581. Therefore, triggered by the detection by the sensor 587 of whether the liquid ejection device 195 is accommodated or not, the ECU 586 may execute the coordinated processing or the like in the foregoing embodiments. In the embodiment shown in FIG. 22, a superabsorbent polymer 583 which absorbs the liquid leaking out from the suction tube 130 or the like is buried in the main body 581. Therefore, the absence of a liquid storage section does not matter when using the stand. In this stand 580, there is no enclosed housing passage and the state of the liquid ejection device 195 can be constantly visually observed. Also, there is no risk of damage to the suction tube 130 and the ejection nozzle or the like due to a collision with the edge of the housing passage. Since a part of the ring-shaped holder 584 is cut out, it is easy to accommodate and remove the liquid ejection device 195. The first to eleventh embodiments can also be carried out with a stand of the shape shown in FIG. 22 or with stands of other shapes.

In the embodiments, the volume of the liquid chamber 112 is changed by the movement of the diaphragm 114, thus ejecting the liquid. However, the volume of the liquid chamber 112 may be changed by a piston instead of the diaphragm 114. Also, instead of using the diaphragm 114, a laser beam may be cast on the liquid inside the liquid chamber to raise the pressure inside the liquid chamber quickly within a short time, thus ejecting the liquid. Alternatively, the liquid may be ejected by the pressure of the feed pump 200, without using the diaphragm 114 or the liquid chamber 112. The liquid ejection is not limited to the pulsed flow ejection but may be continuous flow ejection. Also, while the liquid discharge processing is described as including the initial filling operation and the liquid discharge operation to discharge air bubbles, the liquid discharge operation to prevent the distal end of the nozzle 124 from solidifying may also be carried out. Moreover, the device can also be used as a device for holding other medical instruments such as electric surgical knife, and laser knife. The above embodiments can be carried out singly or two or more of the embodiments can be combined arbitrarily.

## Claims

1. A medical instrument holding apparatus that holds a medical instrument having a functional unit, the apparatus comprising:
an instrument housing unit capable of housing at least a part of the instrument; and
a coordination unit which coordinates with the functional unit when at least a part of the instrument is housed in the instrument housing unit.

2. The medical instrument holding apparatus according to claim 1, wherein a function of the instrument is a function of ejecting a liquid outside from an ejection portion provided on the instrument, and the ejection portion is housed in the instrument housing unit.

3. The medical instrument holding apparatus according to claim 2, wherein the coordination unit is a cleaning device which cleans at least the ejection portion of the liquid of the instrument housed in the instrument housing unit.

4. The medical instrument holding apparatus according to claim 2 or 3, wherein the coordination unit includes a liquid discharge device which discharges the liquid from inside the instrument housing unit.

5. The medical instrument holding apparatus according to claim 2, 3 or 4, wherein the coordination unit includes a high molecular polymer which adsorbs the liquid leaking out of the instrument in the instrument housing unit.

6. The medical instrument holding apparatus according to any one of the preceding claims, wherein the coordination unit includes a signal output unit which outputs a signal outside when the medical instrument is housed in the instrument housing unit.

7. The medical instrument holding apparatus according to any one of the preceding claims, wherein the coordination unit includes an actuation unit which causes a recognition device provided on the side of the medical instrument housed in the instrument housing unit to recognize that a part of the instrument is housed in the instrument housing unit.

8. The medical instrument holding apparatus according to any one of the preceding claims, wherein the coordination unit includes a data unit which communicates data that causes at least one of a type of the medical instrument holding apparatus and an individual to be recognized, with the instrument housed in the instrument housing unit.

9. The medical instrument holding apparatus according to any one of the preceding claims, wherein the coordination unit includes a member which carries out at least one of heating and cooling of the instrument housed in the instrument housing unit.

10. The medical instrument holding apparatus according to any one of the preceding claims, comprising a film which covers an inlet port of the instrument housing unit before the use of the medical instrument holding apparatus.

11. The medical instrument holding apparatus according to any one of the preceding claims, wherein the apparatus is sterilized and packed with the medical instrument.

12. The medical instrument holding apparatus according to any one of the preceding claims, comprising:
a replacement housing unit which houses a part including at least a distal end of a replaceable replacement member provided on the medical instrument, in order to replace the replacement member; and
a detachment unit which detaches the replacement member housed in the replacement housing unit from the instrument.

13. The medical instrument holding apparatus according to claim 12, comprising:
a new replacement member installed on the instrument instead of the detached replacement member; and
a replacement member installing unit which houses the new replacement member in such a way that the new replacement member can be installed on the instrument.
